(19) Europäisches Patentamt
European Patent Office
Office européen des brevets

(11) **EP 1 615 674 B1**

(12) **EUROPÄISCHE PATENTSCHRIFT**

(45) Veröffentlichungstag und Bekanntmachung des Hinweises auf die Patenterteilung:
**12.06.2013 Patentblatt 2013/24**

(51) Int Cl.:
*A61L 15/42* (2006.01)      *A61L 15/28* (2006.01)
*A61L 31/04* (2006.01)      *A61L 31/14* (2006.01)

(21) Anmeldenummer: 04727003.8

(22) Anmeldetag: **13.04.2004**

(86) Internationale Anmeldenummer:
**PCT/EP2004/003850**

(87) Internationale Veröffentlichungsnummer:
**WO 2004/091677 (28.10.2004 Gazette 2004/44)**

(54) **SELBSTHAFTENDES RESORBIERBARES HÄMOSTYPTIKUM**

SELF-ADHESIVE REABSORBABLE HEMOSTYPTIC

HEMOSTYPTIQUE AUTO-ADHESIF RESORBABLE

(84) Benannte Vertragsstaaten:
**AT BE BG CH CY CZ DE DK EE ES FI FR GB GR HU IE IT LI LU MC NL PL PT RO SE SI SK TR**

(30) Priorität: **17.04.2003 DE 10318802**

(43) Veröffentlichungstag der Anmeldung:
**18.01.2006 Patentblatt 2006/03**

(73) Patentinhaber: **Aesculap AG**
**78532 Tuttlingen (DE)**

(72) Erfinder:
• **ODERMATT, Erich, K.**
  **CH-8200 Schaffhausen (CH)**
• **WEGMANN, Jürgen**
  **78333 Stockach (DE)**
• **BLENDER, Bernd**
  **88367 Hohentengen (DE)**
• **DUFFNER, Jürgen**
  **78315 Radolfzell (DE)**

(74) Vertreter: **Patentanwälte**
**Ruff, Wilhelm, Beier, Dauster & Partner**
**Postfach 10 40 36**
**70035 Stuttgart (DE)**

(56) Entgegenhaltungen:
**EP-A- 1 424 085    US-A- 3 868 955**

• **DATABASE EMBASE [Online] ELSEVIER SCIENCE PUBLISHERS, AMSTERDAM, NL; 2002, OKUYAMA H ET AL: "Laparoscopic rectopexy for rectal prolapse in children" XP002291070 Database accession no. EMB-2003038228 & PEDIATRIC ENDOSURGERY AND INNOVATIVE TECHNIQUES 2002 UNITED STATES, Bd. 6, Nr. 4, 2002, Seiten 285-288, ISSN: 1092-6410**

**Beschreibung**

Die Erfindung betrifft ein an menschlichem oder tierischem Gewebe selbsthaftendes resorbierbares Hämostyptikum, im wesentlichen aus mindestens einem, freie Aldehydgruppen tragenden Polymer, dessen Aldehydgruppen mit nukleophilen Gruppen des Gewebes zu reagieren vermögen, wobei das Hämostyptikum in fester, trockener, poröser und saugfähiger Form vorliegt, sowie ein Verfahren zu seiner Herstellung.

In der modernen Chirurgie werden zum Verschließen von inneren und äußeren Wunden im Körpergewebe sowie zum Zusammenfügen zweier getrennter Gewebeteile vor allem in der minimalinvasiven Chirurgie immer häufiger Gewebekleber, wie beispielsweise in der US 6,156,488 oder der DE 101 52 407 beschrieben, eingesetzt. Diese flüssigen Gewebekleber führen jedoch zu Schwierigkeiten bei bestimmten Applikationen, wie bei der Verklebung einer flächigen Wunde, bei gewölbten zu reparierenden Gewebeteilen und bei schwer zugänglichen Wunden sowie beim Verschluss von inneren Wunden bei minimalinvasiven chirurgischen Eingriffen, da die flüssigen Gewebekleber nicht an jede beliebige Stelle der zu verschließenden Wunden bzw. zusammenzufügenden Gewebeteilen gleichmäßig auftragbar sind.

Es ist auch bekannt Collagenvliese zur Blutstillung bei inneren Wunden einzusetzen. Derartige vliesartige Hämostyptika sind beispielsweise unter dem Handelsnamen TachoComb H von NYCOMED und unter dem Handelsnamen Lyostypt der B. Braun-Melsungen AG erhältlich. Diese vliesartigen Hämostyptika besitzen gegenüber den flüssigen Gewebeklebern den Vorteil, auch auf inneren Wunden mit unregelmäßigen Oberflächen gleichmäßig über die Oberfläche der Wunde aufgetragen werden zu können. Um ein Verbleiben auf der Wunde zu gewährleisten und um ein Verrutschen oder ein Abfallen zu verhindern wird im Falle des TachoComb H Collagenvlieses ein Zweikomponentenkleber dem Vliesmaterial bei der Herstellung zugegeben, um eine Gewebeklebewirkung des blutstillenden Vlieses auf der Gewebeoberfläche zu gewährleisten. Derartige blutstillende Collagenvliese ohne eine zumeist auf Fibrinklebern basierende Gewebeklebstoffkomponente besitzen nur eine sehr geringe Haftkraft auf blutenden Gewebeoberflächen. Die Kleberkomponenten bestehen zumeist aus Thrombin und Fibrinogen, wobei Thrombin und Fibrinogen hierbei aktiv in die Blutgerinnungskaskade eingreift. Ein großer Nachteil der Collagen- bzw. Thrombin- und Fibrinogenhämostyptika ist im tierischen, zumeist bovinen, porcinen oder equinen, oder humanen Ursprungs des Collagens, des Thrombins und Fibrinogens zu sehen. Unter dem Hintergrund insbesondere der BSE- und HIV-Problematik ist ein Infektionsrisiko durch die Verwendung von chirurgischem Material tierischen und humanen Ursprungs nicht mehr auszuschließen. Zudem ist bei den derzeit verfügbaren hämostyptischen Vliesen tierischen Ursprungs der Zusatz einer haftungsvermittelnden Komponente in Form eines zumeist Zweikomponentenklebstoffs notwendig, welcher in das Vlies eingearbeitet werden muss. Diese Klebstoffkomponenten in Form von Thrombin und Fibrinogen stellen jedoch einen Eingriff in die Blutgerinnungskaskade dar.

Die europäische Patentanmeldung EP 815 879 der Johnson & Johnson Medical Incorporation beschreibt ein bioabsorbierbares Material, welches aus oxidierten Polysacchariden in Form eines gefriergetrockneten Schwammes zur Hämostase und zur Adhäsionsvermeidung bei chirurgischen Eingriffen angewendet werden kann. Das bioabsorbierbare Material besteht aus einem wasserlöslichen Zellulosederivat, welches zur Carbonsäure oxidierte primäre Alkoholgruppen im Bereich von 3 bis 12 % aufweist. Dieses bioabsorbierbare Material, unter dem Handelsnamen TABOTAMP kommerziell erhältlich, findet vor allem zur Verhinderung von Blutungen nach operativen Eingriffen Verwendung und verfügt daher gemäß seiner zugrundeliegenden Aufgabe über keinerlei Hafteigenschaften gegenüber Gewebe bzw. Gewebeteilen.

Aus der EP 693 291 der United States Surgical Corporation, USA ist eine Wundzusammensetzung in Form eines Pulvers bzw. in Verbindung mit einem Übertragungsvehikel in Form einer Flüssigkeit oder Paste aus einem oxidierten vernetzten Polysaccharid zur Behandlung einer Wundstelle bekannt. Derartige Wundbehandlungszusammensetzungen sind auch unter dem kommerziellen Namen Debrisan zur Absorption von Wundexsudaten und zur Entfernung von Fremdkörpern aus Gewebewunden bekannt. Das in der EP 693 291 eingesetzte oxidierte vernetzte Polysaccharid besitzt zur Carbonsäure oxidierte Monosaccharid-Hydroxylgruppen. Auch diese Zusammensetzung wirft wie auch die flüssigen Gewebekleber die Problematik des gleichmäßigen Auftragens auf unebene und gewölbte Wunden bzw. Gewebe oder Gewebeteile auf. Ein gleichmäßiges Bedecken einer Wunde ist mit einer derartigen pulverförmigen oder flüssigen Zusammensetzung nicht möglich. Daneben ist für den Verschluss der Gewebewunde nach der Wundversorgung durch die pulverförmige Wundbehandlungszusammensetzung ein Wundverschluss durch ein weiteres Hilfsmittel in Form eines Wundschlussbandes notwendig, da die Zusammensetzung selbst keinerlei Klebeeigenschaften aufweist.

Die EP 1 424 085 A1 beschreibt einen blutstillenden Wundverband in Form eines Vlieses oder Gels und seine Verwendung zur blutstillenden Versorgung einer Wunde. Der Wundverband weist ein Substrat auf, das ein aldehydmodifiziertes Polysaccharid aufweist, wobei die Aldehydgruppen des Polysaccharids kovalente Bindungen mit biologischen Materialien, welche Aminogruppen tragen, eingehen können.

Aus der US 3,868,955 sind aldehydische Polysaccharide enthaltende Materialien, insbesondere zur Vermeidung unangenehmer Körpergerüche, bekannt.

Okuyama H. et al. (Laparoscopic rectopexy for rectal prolapse in children, Pediatric Endosurgery and Innovative Techniques, 2002, 6 (4): 285 - 288) beschreibt den Einsatz von Oxycellulose als Klebstoff und Hämostatikum bei der laparoskopischen Rektopexy von rektalen Prolapsen bei Kindern, wobei die Oxycellulose nach dem chirurgischen Eingriff in dem retrorektalen Volumen belassen wird.

Es ist daher wünschenswert ein Hämostyptikum nicht tierischen oder humanen Ursprungs, welches unabhängig von der Art und Oberfläche der Wunde gleichmäßig aufgebracht werden kann und keinerlei zusätzliche Hilfsmittel zur Fixierung auf der Wunde bzw. zum Verschluss der Wunde bedarf, sowie ein Verfahren zu seiner Herstellung bereit zu stellen.

Die Erfindung löst diese Aufgabe durch ein an menschlichem oder tierischem Gewebe selbsthaftendes resorbierbares Hämostyptikum, im wesentlichen aus mindestens einem, freie Aldehydgruppen tragenden Polymer, dessen Aldehydgruppen mit den nukleophilen Gruppen des Gewebes zu reagieren vermögen, wobei das Hämostyptikum in fester, poröser und saugfähiger Form vorliegt gemäß dem unabhängigen Anspruch 1. Vorteilhafte Weiterbildungen sind in den Ansprüchen 2 bis 14 und 16 bis 19 beschrieben.

Eine weitere Lösung besteht in einem Verfahren zur Herstellung eines Hämostyptikums gemäss dem unabhängigen Anspruch 15.

Der Wortlaut sämtlicher Ansprüche wird durch Bezugnahme zum Inhalt der Beschreibung gemacht.

Das erfindungsgemäße Hämostyptikum liegt in der Regel in trockener Form vor. Er kann aber auch in feuchter Form vorliegen. Mit Vorteil besitzt es eine faserige Struktur und liegt insbesondere als Vlies vor.

Ein Vorteil des erfindungsgemäßen Hämostyptikums besteht darin, dass es ohne weitere Fixierungen oder Klebstoffkomponenten auf der Wundoberfläche haftet und durch seine poröse und saugfähige Form einen schnellen Verschluss einer stark blutenden Wunde ermöglicht. Dabei ist das Hämostyptikum, sobald es einmal auf die Wunde aufgebracht ist, nicht mehr verschiebbar. Ein weiterer Vorteil besteht in der vorzugsweise festen und trotzdem elastisch komprimierbaren Form des Hämostyptikums, welches auch bei minimalinvasiven chirurgischen Eingriffen an schwer zugänglichen inneren Wunden in einer gleichmäßigen Dicke über die gesamte Wundoberfläche aufgebracht werden kann.

Ein weiterer Vorteil besteht im nicht bovinen oder humanen Ursprung des Ausgangsmaterials des erfindungsgemäßen Hämostyptikums, wodurch eine mögliche Infektion im Hinblick auf die Creutzfeldt-Jakob- und BSE- sowie HIV-Problematik ausgeschlossen werden kann.

Das erfindungsgemäße Hämostyptikum besteht im wesentlichen aus mindestens einem, freie Aldehydgruppen tragenden Polymer, dessen Aldehydgruppen mit den nukleophilen Gruppen, insbesondere Aminogruppen, SH-Gruppen und OH-Gruppen, des menschlichen oder tierischen Gewebes zu reagieren vermögen. Das Hämostyptikum ist an menschlichem oder tierischem Gewebe selbsthaftend, d.h. ohne weitere Fixierungsmittel auf einer Wundoberfläche anbringbar. Es ist resorbierbar, d.h. es wird im menschlichen oder tierischen Körper nach einer gewissen Zeit vollständig abgebaut.

Das Hämostyptikum liegt in fester, insbesondere trockener Form vor und besitzt vorzugsweise eine schwammartige oder faserartige poröse Struktur. Das Hämostyptikum weist stark saugende Eigenschaften auf, wodurch eine hohe Konzentration von Blutplättchen an der Oberfläche und im Inneren des Hämostyptikums erfolgt.

Das Hämostyptikum bildet eine Haftverbindung, insbesondere durch Iminbindungen, zwischen den Aldehydgruppen des Polymers und den Aminogruppen des Blutes, Serums und vor allem des umgebenden Körpergewebes. Bei diesen Iminbindungen handelt es sich um reversible kovalente Bindungen, welche stärker als reine ionische Bindungen sind und daher ein gutes Haften zwischen Hämostyptikum und Gewebe ermöglichen. Im Falle von SH- bzw. OH-Gruppen bildet das erfindungsgemäße Hämostyptikum Haftverbindungen in Form von Acetal- bzw. Thioacteal-Bindungen aus, die sich entsprechend der Iminbindungen verhalten. Durch das erfindungsgemäße Hämostyptikum wird nicht nur das Blut an der Wundstelle durch die saugende Wirkung gebunden, das Hämostyptikum wirkt durch die beginnende Gelierung auch als mechanische Barriere, die den Blutaustritt verhindert. Außerdem verschließt es durch seine Klebeeigenschaften die zu reparierende Gewebestelle und verstärkt durch diesen Verschluss der Wunde zusätzlich seine blutstillende Wirkung und verhindert dadurch auch mögliche Verwachsungen mit benachbartem Gewebe. Durch das erfindungsgemäße Hämostyptikum wird das Blut mit rein physikalisch chemischen Mitteln gebunden, ohne dabei wie im Falle von Thrombin und Fibrinogen in die Blutgerinnungskaskade einzugreifen. Beim erfindungsgemäßen Hämostyptikum handelt es sich nicht um ein Arzneimittel.

Der Dextranpolyaldehyd verfügt über Vernetzungen, über welche sich die Stabilität und Härte des Hämostyptikums einstellen lassen. Die Degradationsdauer lässt sich ebenfalls durch Zusatz von Vernetzern einstellen bzw. verlangsamen. Der Dextranpolyaldehyd des Hämostyptikums liegt vorzugsweise in unvernetzter Form vor.

Es ist auch denkbar, dass das Hämostyptikum Zusatzstoffe, insbesondere Weichmacher, enthält. In einer Ausführungsform enthält das Hämostyptikum Glycerin als Weichmacher. Es ist weiter denkbar, dass das Hämostyptikum pharmakologisch wirksame Substanzen enthält, die aus dem Hämostyptikum an das umgebende Gewebe und Körperflüssigkeiten abgegeben werden.

Das Hämostyptikum kann auch einen Zusatz zur Erhöhung der Saugkraft enthalten. Unter Saugkraft wird hierbei sowohl die Geschwindigkeit der Flüssiglceifisaufnahme als auch die absolut aufgenomme Menge verstanden.

Dadurch wird die Zeit zur Stillung einer Blutung zusätzlich verkürzt und das Risiko des Durchblutens des Hämostyptikum vermindert. In einer besonderen Ausführungsform wird als Zusatz zur Erhöhung der Saugfähigkeit Carboxymethylcellulose (CMC) eingesetzt.

Das erfindungsgemäße Hämostyptikum kann elastisch komprimierbar sein. Das Hämostyptikum wird in einer Ausführungsform zur Verbesserung der Elastizität nach der Herstellung mittels einer Pressvorrichtung, insbesondere auf eine Stärke von 0,3-0,4 mm, im wesentlichen reversibel verpresst. Durch das Verpressen wird die Flexibilität des Hämostyptikum deutlich erhöht. Der Abknickwinkel des Hämostyptikums beim Knicken aus der Ebene in trockenem Zustand beträgt beim unverpressten Hämostyptikum mit Vorteil 10° und beim verpressten Hämostyptikum mit Vorteil 30°. Dadurch ist ein Anmodellieren des Hämostyptikums auf der Wunde sehr viel besser und leichter möglich, ohne dass das Hämostyptikum bricht.

In einer Ausführungsform besitzt das Hämostyptikum die Form eines dreidimensionalen Körpers und liegt vorzugsweise plattenförmig vor.

In einer besonderen Ausführungsform liegt das Hämostyptikum in faserförmiger Form, vorzugsweise in Form eines, insbesondere dreidimensionalen, Vlieses mit einem faserartigen Aufbau vor, welches eine Gesamtoberfläche besitzt, die um ein mehrfaches größer ist als die Fläche des Vlieses.

Es ist auch denkbar, dass das Hämostyptikum in Form eines offenporigen Schaumes vorliegt. Dieser Schaum besitzt im Verhältnis zur Fläche des Hämostyptikums eine um ein vielfaches größere innere Oberfläche.

Gemäß weiterer Ausführungsformen liegt das Hämostyptikum in Form eines Granulates oder eines Pulvers aus saugfähigen Partikeln vor.

In einer andern Ausführungsform liegt das Hämostyptikum in Form einer Folie oder Membran vor. Die Folie oder Membran kann durch Pressen oder Walzen eines Schaumes oder Lyophilisats oder durch gießen einer Lösung und anschließendem Trocknen der Lösung erhalten werden. Möglich ist auch, eine Folie durch Rakeln einer Dextranaldehydlösung herzustellen.

Das erfindungsgemäße Hämostyptikum kann in verschiedenen dreidimensionalen Formen vorliegen. Es ist denkbar, dass das Hämostyptikum in Form eines plattenartigen Patches, oder in Form eines Ringes, vorliegt. Es ist auch denkbar, dass das Hämostyptikum rohrförmig elastisch oder formstabil ausgebildet ist.

Vorteilhafterweise ist der Dextranpolyaldehyd und vorzugsweise das gesamte Hämostyptikum wasserlöslich. Damit ist das erfindungsgemäße Hämostyptikum in den verschiedenartigen wasserhaltigen Körperflüssigkeiten vollständig auflösbar. Die Zeit des Auflösens im Körper kann durch den Grad der Vernetzung eingestellt werden. Das Hämostyptikum kann auch in feuchter Form und insbesondere in besonderen Fällen in Lösung, vorzugsweise in einer Einkammerspritze, oder in Form eines Gels, vorzugsweise in einer Zweikammerspritze, vorliegen und vorzugsweise in dieser Form eingesetzt werden. Die Besonderheiten der festen, trockenen und saugfähigen Form entfallen dann.

Bei dem Aldehydgruppen tragenden Polymer handelt es sich um ein oxidiertes, insbesondere bioabsorbierbares Polysaccharid, wobei es sich bei dem Polysaccharid um Dextranpolyaldehyd handelt.

Der Dextranpolyaldehyd besitzt einen Anteil an zum Aldehyd oxidierten Glukoseeinheiten von mindestens 20 %, vorzugsweise von 35 -100 % und insbesondere zwischen 60 und 80 %. Durch einen hohen Anteil an zum Aldehyd oxidierten Glukoseeinheiten wird durch die Vielzahl von kovalenten Bindungen, insbesondere Iminbindungen, eine starke Haftbindung des Hämostyptikums an das Körpergewebe erzielt.

Gemäß einer Ausführungsform ist das erfindungsgemäße Hämostyptikum durch Lyophilisation einer Lösung des mindestens einen Dextranpolyaldehyds erhältlich. Es ist auch denkbar, dass das Hämostyptikum aus einer Lösung des mindestens einen Dextranpolyaldehyds durch Aufschäumen erhältlich ist. Eine deutlich größerer Oberfläche und luftigere Struktur des Hämostyptikums ist durch Zugabe von zerstoßenem Eis erhältlich.

Gemäß einer Ausführungsform ist das Hämostyptikum aus einer 0,5 %i-gen bis 20 %igen, vorzugsweise 1 %igen bis 15 %igen, Lösung des mindestens einen Dextranpolyaldehyds erhältlich. In einer besonders bevorzugten Ausführungsform ist das Hämostyptikum aus einer 1 %igen bis 10 %igen, insbesondere 2,5 %igen, Lösung des mindestens einen Dextranpolyaldehyds erhältlich.

Das erfindungsgemäße Hämostyptikum kann aufgrund seiner schwammartigen Struktur und Porosität und seines hydrophilen Charakters mindestens das 30-fache seines Eigengewichtes an Flüssigkeit aufnehmen. In einer besonderen Ausführungsform, bei der das Hämostyptikum zusätzlich nach der Herstellung verpresst ist, kann das Quellvermögen bzw. die Wasseraufnahemfähigkeit reduziert sein. Diese reduzierung der Wasseraufnahme auf max. das 20 fache des Eigengewichtes ist durch den Zusatz zur Erhöhung der Saugkraft teilweise auszugleichen und immer noch ausreichend, um stark blutenden Wunden zuverschliessen. Die durch das Verpressen erhöhte Elastizität des Hämostyptikums und die damit verbundene bessere Anpassung an die Oberfläche gleicht diesen Effekt des Verpressens annähernd wieder aus. Außerdem ist das Hämostyptikum in der Lage, mindestens das 3-fache seines Eigengewichtes an Hämoglobin aufzunehmen.

Das mindestens eine, Aldehydgruppen tragende Polymer ist vor seiner Anwendung teilweise mit einem Vernetzungsmittel, vorzugsweise Chitosan, vernetzt. Es sind jedoch auch andere Vernetzungsmittel in Form von bifunktionellen Aminen, insbesondere die Aminosäuren Lysin, Ornitin, Arginin oder Triethylenglycoldiamin, von multifunktionellen Aminen, insbesondere die Polyaminosäure Polylysin, von bi- bzw. multifunktionellen SH- oder NH$_2$-Gruppen haltigen Molekülen, insbesondere Cystein oder Polycystein, oder von bi- bzw. multifunktionellen Thiolen sowie Peptiden denkbar.

In einer besonderen Ausführungsform besitzt das Hämostyptikum eine mindestens einseitig strukturierte Oberfläche. Die strukturierte Oberfläche verbessert die Haftung des Hämostyptikums auf dem Gewebe. Die Strukturierung kann einseitig oder beidseitig aufgebracht werden. Es sind verschieden Arten der Strukturierung denkbar, wie eine quadratische, zackige, geflochtene, gewebte oder spiralförmige Struktur. Durch die Strukturierung wird die mechanische Reibung zwischen Gewebe und Hämostyptikum durch die vergrößerte Oberfläche erhöht und das Hämostyptikum hält nach der Applikation besser an der angebrachten Position. Es ist weiterhin möglich eine Struktur in Form von Perforationen, insbesondere durch Ausstanzen oder durch Aufpressen eines Nadelbretts, auf das Hämostyptikum aufzubringen. Dabei wird die primär geschlossene Oberfläche leichter für die aufzunehmende Flüssigkeit zugänglich gemacht und sowohl die Zeit zur Blutstillung als auch die Saugfähigkeit allgemein erhöht.

Das Hämostyptikum ist vorzugsweise bereits nach wenigen Tagen durch Zellen besiedelbar. So wachsen beispielsweise Leberzellen bereits nach 7 Tagen in die Struktur des Hämostyptikums ein. Dadurch ist eine schnelle Heilung der Wunde und eine Wiederherstellung der vollen Funktionalität des Gewebes gewährleistet.

Das erfindungsgemäße Hämostyptikum liegt vorzugsweise sterislisiert, insbesondere in einer sterilisierten Verpackung, vor.

Die Erfindung umfasst weiterhin ein Verfahren zur Herstellung eines Hämostyptikums, welches die Überführung eines sich in Lösung und/oder im Gelzustand befindlichen Dextranpolyaldehyds, das teilweise mit einem Vernetzungsmittel vernetzt ist, durch Lyophilisierung in eine feste trockene Form umfasst. Als Lösungsmedium wird bevorzugt Wasser oder eine wässrige CaCl$_2$-Lösung verwendet.

Eine Strukturierung kann entweder durch entsprechend strukturierte Lyophilisationsschalen oder durch ein Prägen im Anschluss an die Herstellung des Vlieses auf mindestens eine Oberfläche aufgebracht werden.

Das Hämostyptikum kann für eine vorzugsweise innere Anwendung in einem menschlichen oder tierischen Organismus, insbesondere für Wunden, eingesetzt werden.

In einer vorteilhaften Ausführung kann das Hämostyptikum für den Verschluss von vorzugsweise inneren Wunden eingesetzt werden.

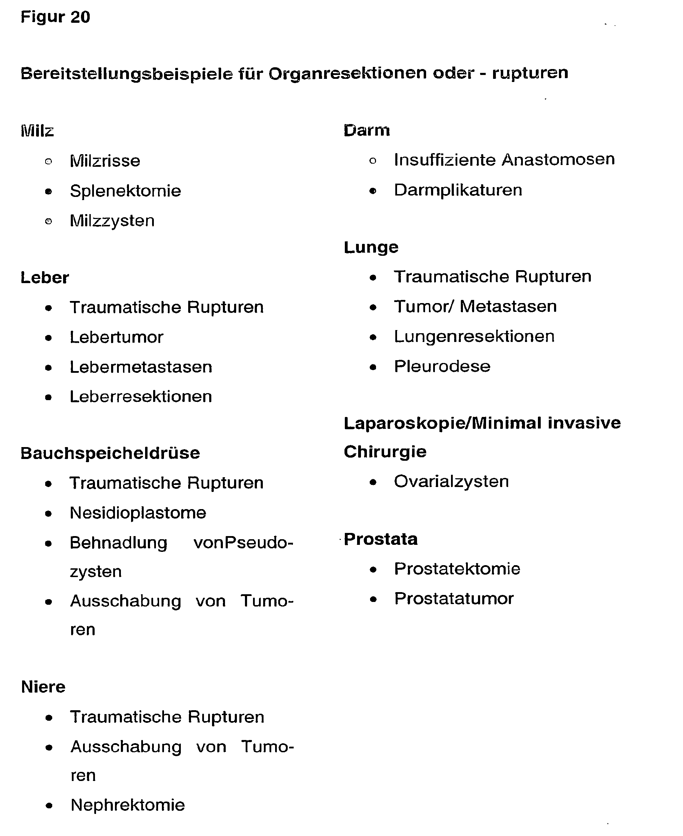

Ein anderer Aspekt umfasst die Bereitstellung des Hämostyptikums bei einer Organresektion oder Ruptur, insbesondere bei der Leber, Niere, Milz oder Bauchspeicheldrüse. Weitere Anwendungsmöglichkeiten siehe Figur 20.

Ein weiterer Aspekt umfasst die Bereitstellung des Hämostyptikums in Form eines Ringes für Anastomosen.

Ein andere Aspekt umfasst die Bereitstellung des Hämostyptikums in Form eines Vlieses, einer Membran oder einer Folie zur Adhäsionsprophylaxe oder als Barriere.

Im folgenden wird die vorliegende Erfindung durch ausführliche Beschreibung einer besonderen Ausführungsform sowie durch Figuren erläutert. In dieser Ausführungsform können einzelne Merkmale der Erfindung allein oder in Kombination mit anderen Merkmalen verwirklicht sein. Die beschriebene besondere Ausführungsform dient zur Erläuterung und zum besseren Verständnis der Erfindung und ist in keiner Weise einschränkend zu verstehen.

Figurenbeschreibung

Figur 1 zeigt eine Vergrößerung eines Dextranaldehydvlieses aus einer einprozentigen Dextranaldehydlösung

Figur 2 zeigt eine Vergrößerung eines Dextranaldehydvlieses aus einer 2 %igen Dextranaldehydlösung

Figur 3 zeigt eine Vergrößerung eines Dextranaldehydvlieses aus einer 3,5 %igen Dextranaldehydlösung

Figur 4 zeigt eine Vergrößerung eines Dextranaldehydvlieses aus einer 5 %igen Dextranaldehydlösung

Figur 5 zeigt eine Vergrößerung eines Dextranaldehydvlieses aus einer 7,5 %igen Dextranaldehydlösung

Figur 6 zeigt die Ergebnisse eines Lee White Clotting Tests

Figur 7 zeigt eine Leberresektion

Figur 8 zeigt die Versiegelung nach einer Leberresektion

Figur 9 zeigt die Traumatisierung einer Leber durch Kreuzschnitt

Figur 10 zeigt die Stillung der traumatisierten Leber aus Figur 9

Figuren 11 bis 14 zeigen die Verwendung des Hämostyptikums in Form eines Anastomosenringes

Figur 15 zeigt das Hämostyptikum in Form eines Vlieses

Figur 16a zeigt das Hämostyptikum in Form eines Anastomosenringes, Figur 16b zeigt eine Detailvergrößerung aus Figur 19a.

Figur 17 zeigt den Einsatz einer Dextranaldehydmembran

Figur 18 zeigt die Synthese eines Spacers

Figur 19 zeigt die Anbindung eines Spacers

Figur 20 zeigt die Bereitstellungsmöglichkeiten des Hämostyptikums bei einer Organresektion oder Ruptur.

Figur 21 zeigt eine durch Kreuzschnitt traumatisierte Leber

Figur 22 zeigt das Anmodellieren eines Hämostyptikums

Figur 23 zeigt die traumatisierte Leber nach Versorgung mit dem Hämostyptikum

Figur 24 zeigt einen Schnitt durch die traumatisierte Leber

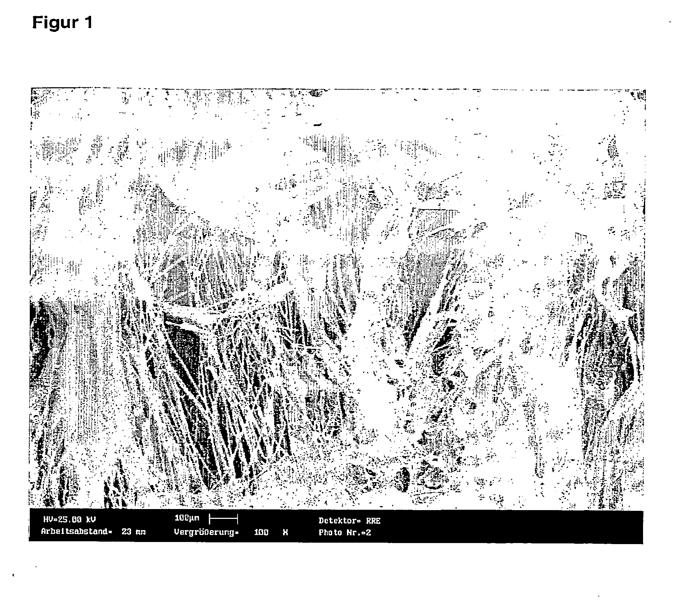

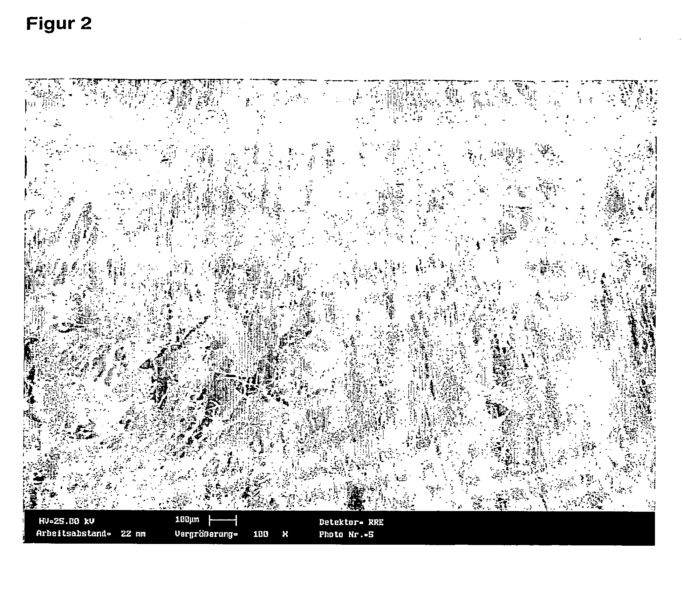

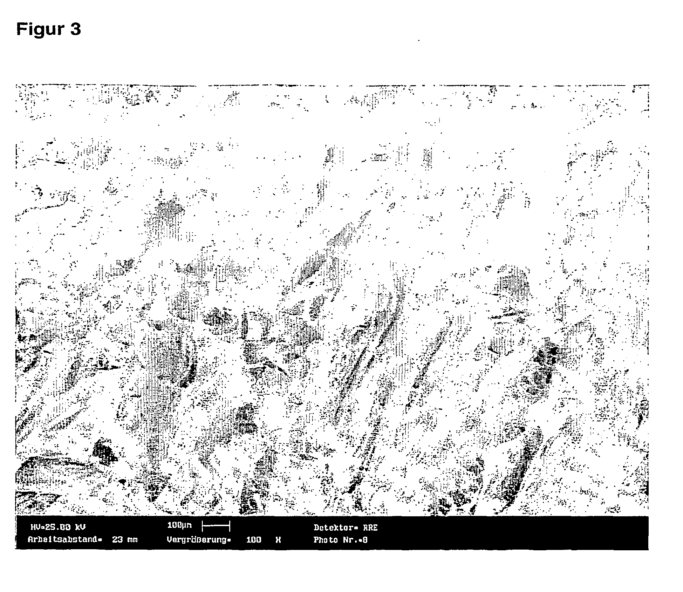

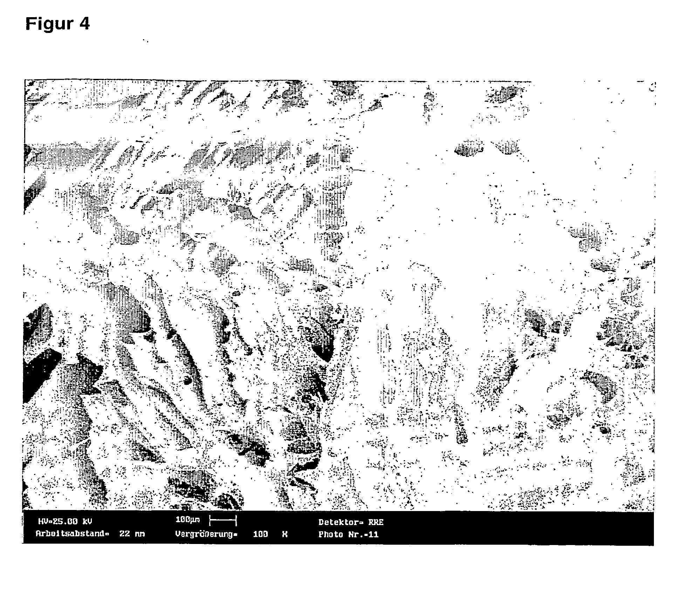

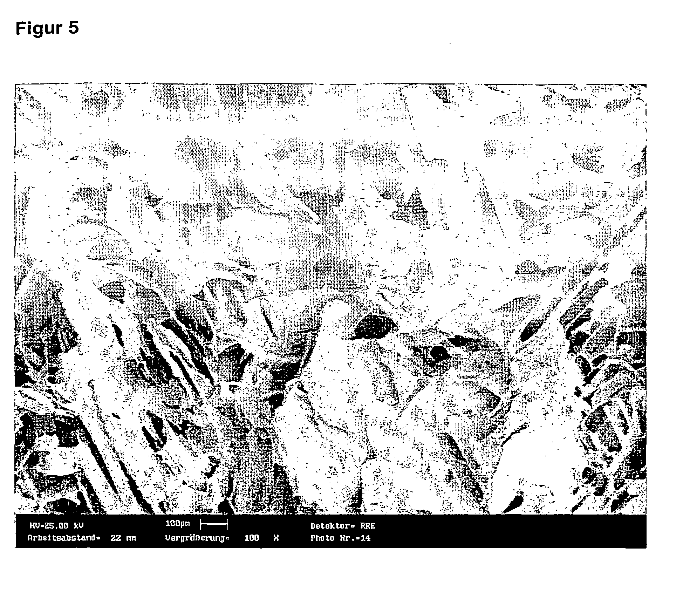

Die Figuren 1 bis 5 zeigen jeweils 100-fache Vergrößerungen der Dextranaldehydvliesstruktur hergestellt aus Dextranaldehydlösungen der Konzentrationen 1 %, 2 %, 3,5 %, 5 % und 7,5 % entsprechend der Vliese 2 bis 6 in Tabelle 1. Mit zunehmender Konzentration der Dextranaldehydlösung wird die Struktur des Dextranaldehydvlieses bei gleicher Füllhöhe der zu lyophilisierenden Lösung deutlich dichter und zeigt im Bereich von 1 % bis 2 % eine faserartige Struktur. Ab einer Dextranaldehydkonzentration von 3,5 % bis 7,5 % sind in zunehmendem Maße eine schwammartige Struktur mit röhrenförmigen und Kavitäten bildenden Strukturen zu erkennen.

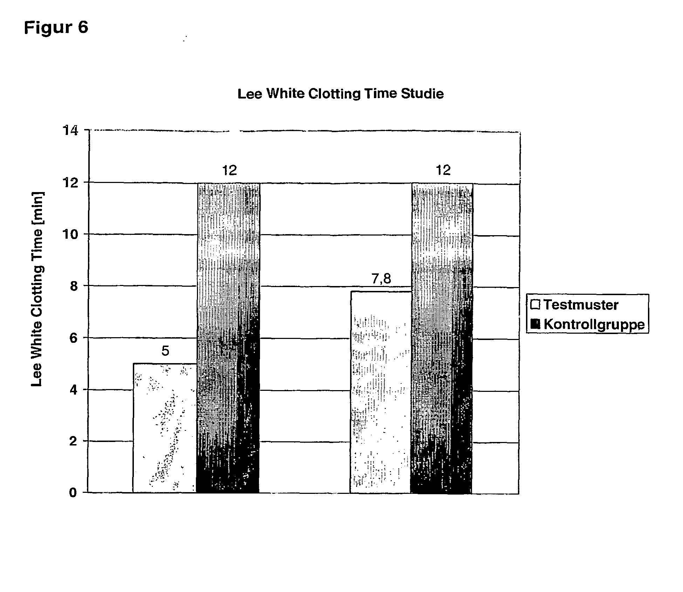

Figur 6 zeigt die Ergebnisse einer Lee White Clotting Time Studie zur Gerinnung einer 15 %igen wässrigen Dextranaldehydlösung und eines Dextranaldehydvlies (hergestellt aus 2 %iger wässriger Dextranaldehydlösung). Sowohl für die Dextranaldehydlösung als auch für das Dextranaldehydvlies ist das Ergebnis mit der jeweiligen Testsubstanz (schraffiert) und die parallele Kontrollreaktion ohne Testsubstanz (ohne Schraffur) dargestellt. Die Lee White Clotting Time betrug für die 15 %ige Dextranaldehydlösung 5 Minuten und für das Dextranaldehydvlies, hergestellt aus 2 %iger wässriger Dextranaldehydlösung, 7,8 Minuten. Die Gerinnungszeit für die Kontrollreaktionen ohne Testsubstanzen betrugen in beiden Fällen 12 Minuten, so dass für das Dextranaldehydvlies eine Verkürzung der Blutgerinnungszeit von ca. 30 % erzielt werden konnte.

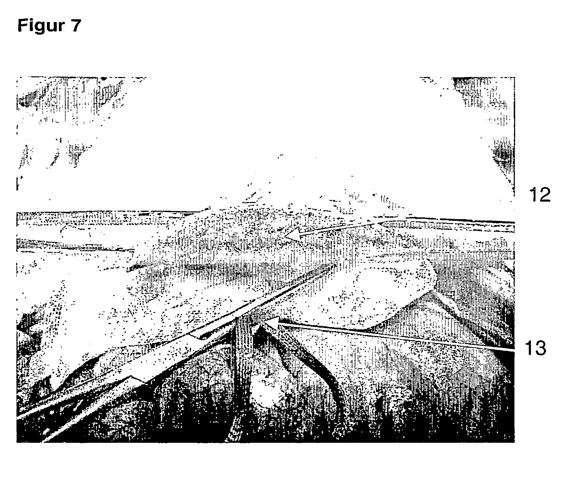

Figur 7 zeigt eine Leberreselction an einer Schweineleber, bei der die Blutversorgung durch ein Pringle-Manöver und Parenchymklemme unterbrochen wurde.

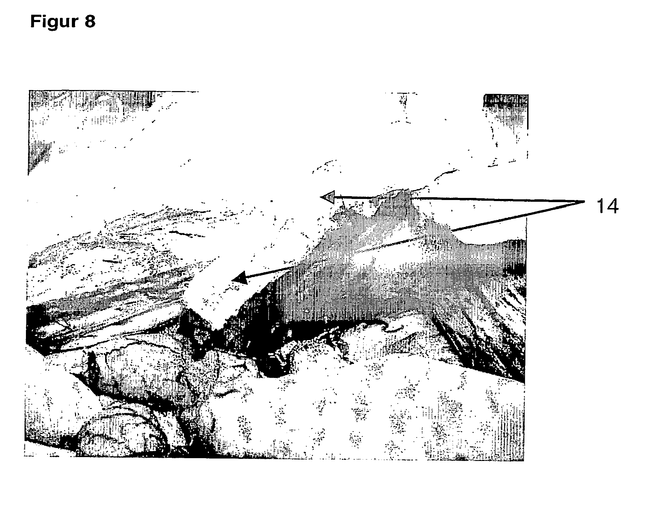

Figur 8 zeigt die im Anschluss an diese Leberresektion (s. Figur 7) durchgeführte Fixierung der Lebergefäße mit Nahtmaterial und anschließende Versiegelung der Gefäße mit dem Polyaldehydvlies.

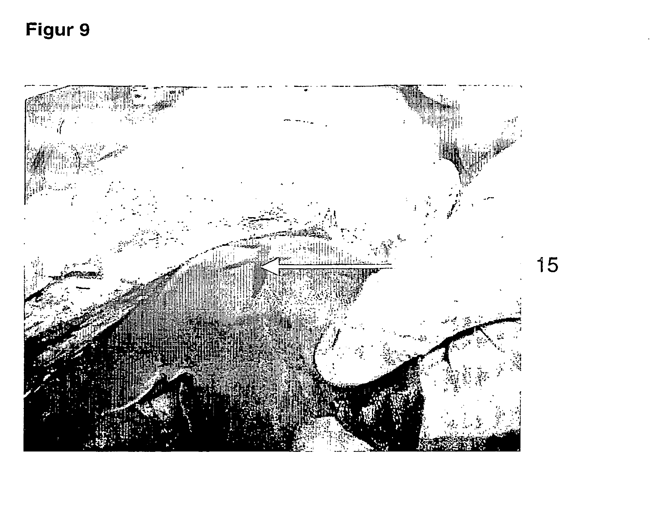

Figur 9 zeigt die Traumatisierung einer Schweineleber durch einen Kreuzschnitt von 2 x 3 cm Länge und 5 cm Tiefe bei intakter Blutversorgung der Leber.

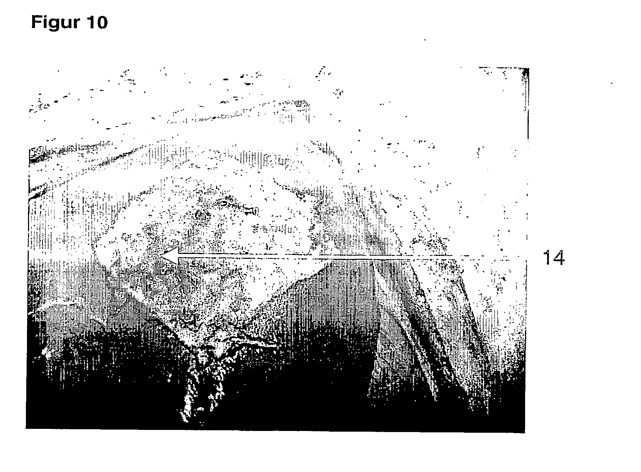

Figur 10 zeigt die Versorgung des Kreuzschnittes (s. Figur 9) bei intakter Blutversorgung der Leber. Durch Anfeuchten eines Polyaldehydvlieses mittels einer befeuchteten Kompresse zur Stillung der Leberblutung. Die Blutung der traumatisierten Leber konnte hierbei durch das angefeuchtete Vlies vollständig zum Stillstand gebracht werden.

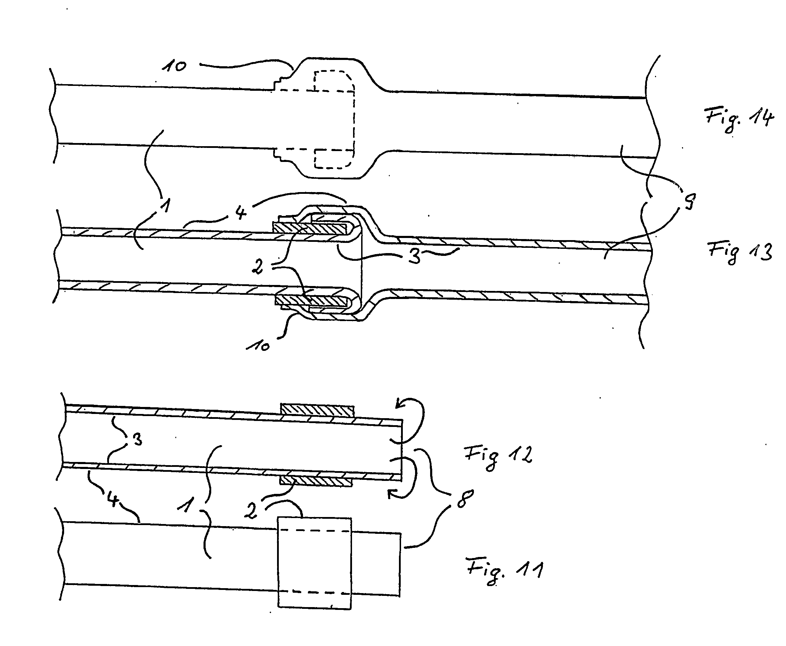

Figur 11 bis Figur 14 zeigen die Verwendung des Hämostyptikums als Anastomosenring. Hierbei liegt das Hämostyptikum in Form eines rohrförmigen Teiles vor.

Figur 11 zeigt einen ersten Blutgefäßabschnitt 1 mit dem zu verbindenden freien Ende 8, bei dem auf die Außenseite 4 des Blutgefäßabschnittes das rohrförmige Hämostyptikum 2 aufgeschoben wird, so dass es zum freien Ende 8 beabstandet ist.

Figur 12 zeigt einen Querschnitt durch den ersten Blutgefäßabschnitt 1, über welchen beabstandet zum freien Ende 8 ein rohrförmiges Hämostyptikum 2 übergestülpt ist. Die beiden Pfeile zeigen an, wie das freie Ende 8 des ersten Blutgefäßabschnittes 1 nach außen über das rohrförmige Hämostyptikum 2 gestülpt wird und dadurch die Intima/Innenseite 3 des Blutgefässes nach außen gekehrt wird.

Figur 13 und Figur 14 zeigen das von außen über das Ende des ersten Blutgefässes 1 gestülpte freie Ende 10 des zweiten Blutgefäßabschnittes 9, wobei die beiden Innenseiten 3 des ersten und zweiten Blutgefäßabschnittes 1, 9 aufeinander zu liegen kommen. Nachdem, wie in Figur 11 und 12 dargestellt, das Hämostyptikum in Form eines Anastomosenringes 2 über das erste Gefäß 1 gezogen wurde, wird das freie Ende wie durch die beiden Pfeile in Figur 12 angedeutet, nach außen über den Anastomosenring 2 übergestülpt, so dass ein Teil des Anastomosenringes mit dem freien Ende 8 des ersten Blutgefäßabschnittes 1 bedeckt und dadurch die Innenseite 3 des ersten Blutgefäßes 1 nach außen gekehrt ist. Anschließend wird ein zweites Blutgefäß 9 über das umgestülpte Ende des Blutgefäßes 1 gezogen und zwar so weit, dass das freie Ende 10 des zweiten Blutgefäßabschnittes 9 über den vom freien Ende 8 des ersten Blutgefäßabschnittes 2 noch nicht bedeckten Teiles des Anastomosenringes 2 geführt wird. Durch das Übereinanderlegen des ersten und zweiten Blutgefäßabschnittes im überlappenden Bereich ergibt sich ein Kontakt der beiden Gefäßinnenseiten zueinander, welcher nach dem Eingriff zu einem schnellen und komplikationslosen Zusammenwachsen der beiden Blutgefäßabschnitte führt und eine kompakte Ausbildung der Intima fördert.

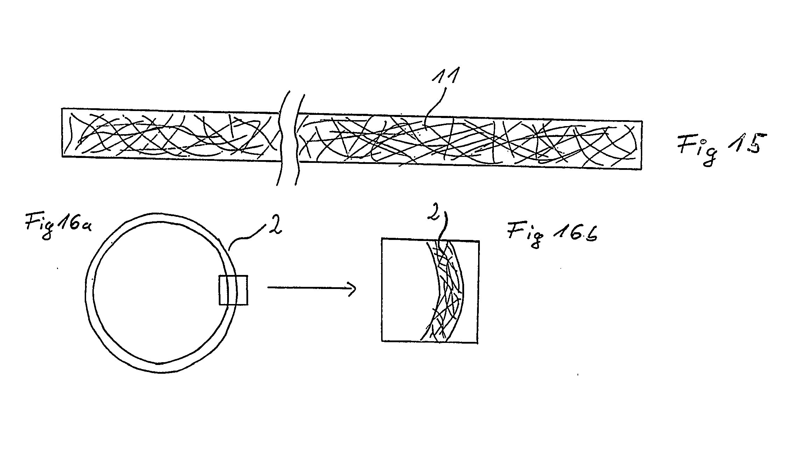

Figur 15 zeigt eine Aufsicht auf den Querschnitt eines dreidimensionalen plattenartigen Hämostyptikums in Form eines Vlieses 11, welches aus einer Dextranaldehydlösung lyophilisiert wurde. In der Aufsicht sind die faserartigen Strukturen des lyophilisierten Dextranaldehyds ersichtlich, die ungeordnet die gesamte Fläche des Hämostyptikum ausfüllen. Die Höhe des Vlieses wird hierbei durch die Füllhöhe der Dextranaldehydlösung vor der Lyophilisierung bestimmt und verändert sich im Verlauf der die Lyophilisierung der Dextranaldehydlösung nicht. Der Anteil an faserartigem Dextranaldehyd in dieser Aufsicht zeigt deutlich den hohen Anteil an Hohlräumen zwischen den faserartigen Dextranaldehydstrukturen, wodurch die hohe Saugfähigkeit des Vlieses bedingt ist.

Figur 16 zeigt das Hämostyptikum in Form eines Anastomosenrings, dessen Verwendung in den Figuren 11 bis 14 dargestellt ist. Figur 16a zeigt die Aufsicht auf den Querschnitt durch das ringförmige Hämostyptikum 2. Figur 16b zeigt eine Detailvergrößerung aus Figur 16a, worin deutlich die faserartigen Dextranaldehydstruktur des Anastomosenringes 2 ersichtlich ist.

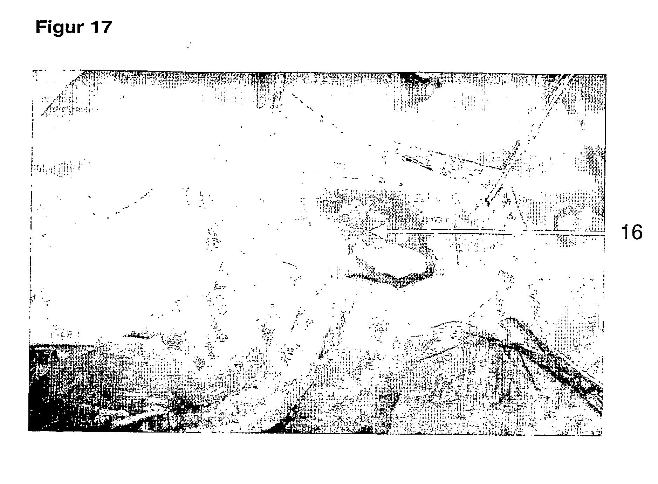

Figur 17 zeigt den Einsatz einer Dextranaldehydmembran 16 als Adhäsionsprophylaxe an einer traumatisierten Bauchwand eines Kaninchens (Rabbit Side Wall Modell).

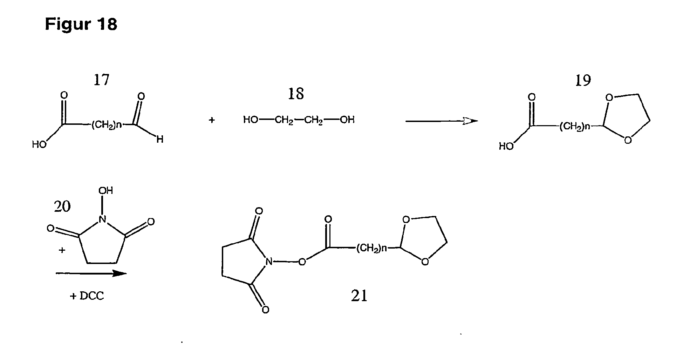

Figur 18 zeigt die Synthese eines Spacers zum Einfügen zwischen dem Polymer und der Aldehydgruppenfunktion (siehe auch Beispiel)

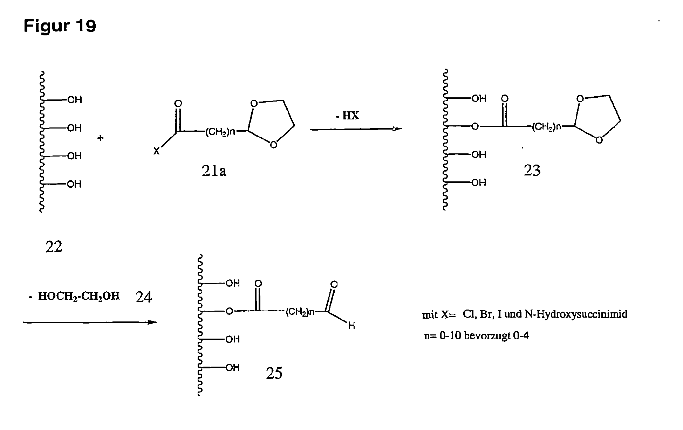

Figur 19 zeigt die Anbindung eines Spacers zunächst an das Polymer und die anschließende Darstellung der Aldehydfunktion (siehe Beispiel)

Figur 20 zeigt die Bereitstellungsmöglichkeiten des Hämostyptikums bei einer Organresektion oder Ruptur.

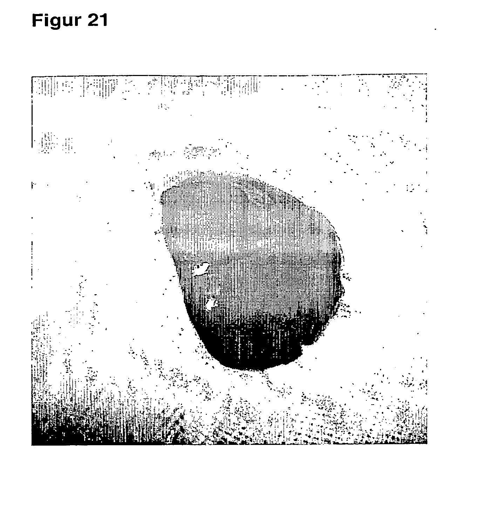

Figur 21 zeigt eine durch Kreuzschnitt traumatisierte Leber einer Ratte.

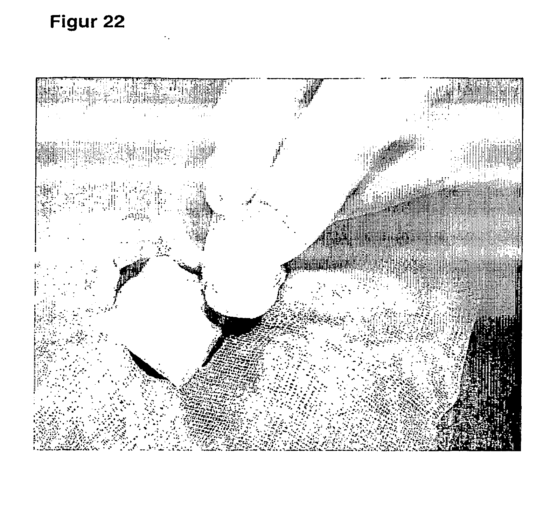

Figur 22 zeigt das Anmodellieren eines gepresssten Hämostyptikums gemäss Beispiel 7 auf die traumatisierte Leber aus Abbildung 21.

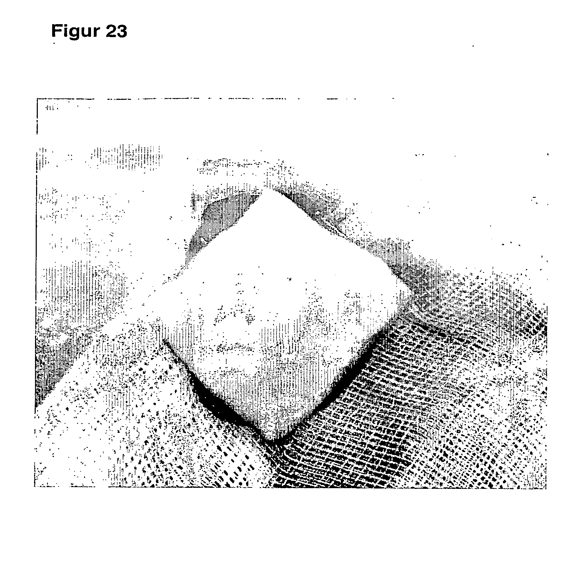

Figur 23 zeigt die traumatisierte Leber nach Versorgung und erfolgreicher Blutstillung mit dem Hämostyptikum gemäss Beispiel 7.

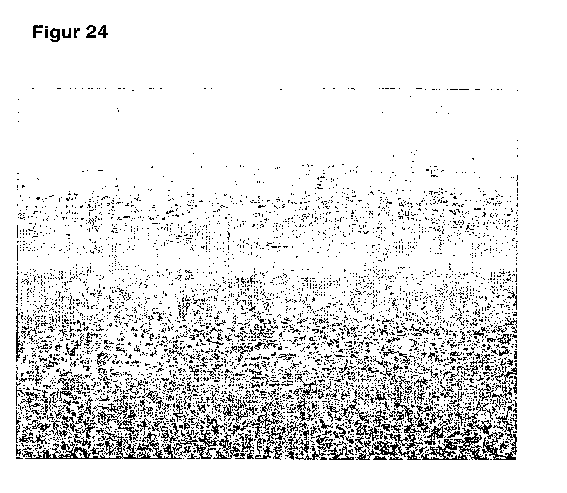

Figur 24 zeigt einen histopathologischer Schnitt durch die traumatisierte Leber aus Abbildung 21, welche 7 Tage nach der OP entnommen wurde. Dabei sind (1) Leberparenchym, (2) Bindegewebskapsel, (3) Leberregenerationsgewebe, (4) Vliesmaterial, (5) äußere Bindegewebsschicht.

<u>Beispiele</u>

1. Herstellung eines Hämostyptikums in vliesartiger Form:

Dextranaldehyd wird in bidestilliertem Wasser bei 50°C gelöst. Die Lösung wird in Schalen gegossen und lyophilisiert, wobei die Füllhöhe der Schalen mit der Dextranaldehydlösung die Dicke des hämostyptischen Vlieses bestimmt. Es wurden Vliese aus Lösungen mit unterschiedlicher Konzentration an Dextranaldehyd hergestellt. Das Flächengewicht der Vliese lässt sich durch die Konzentration und Dicke des Vlieses einstellen. Aus der 0,5 %igen Dextranaldehydlösung wurde durch Lyophilisierung kein zusammenhängendes Vlies erzielt. Das Lyophilisat bestand aus einzelnen Bruchstücken. Mit zunehmender Konzentration an Dextranaldehyd wird die Struktur des faserartigen hämostyptischen Vlieses dichter (s. Figur 1 bis Figur 5). Mit zunehmender Konzentration und Dichte der Struktur werden immer härtere und druckstabilere Vliese erzielt, die dabei an Elastizität verlieren.

Tabelle 1: Variation der Dextranaldehydkonzentration

| Vliesnummer | Konzentration der Ausgangslösung (w/v) | Füllhöhe der Schalen (cm) | Vliesdicke (cm) | Flächengewicht (g/m²) |
|---|---|---|---|---|
| 1 | 0,5 % | 0,6 | Nicht bestimmbar, Einzelne Bruchstücke | Nicht bestimmbar |
| 2 | 1 % | 0,6 | Ungleichmäßige Dicke | 59 |
| 3 | 2 % | 0,6 | 0,6 | 140 |
| 4 | 3,5 % | 0,6 | 0,6 | 240 |
| 5 | 5 % | 0,6 | 0,6 | 340 |
| 6 | 7,5 % | 0,6 | 0,6 | 527 |

2. Lee White-Gerinnungstest:

Mit Hilfe des Lee White-Gerinnungstests wurden folgende Testmuster auf ihre hämostyptischen Eigenschaften untersucht: 15 %ige wässrige Dextranaldehydlösung und Dextranaldehydvlies (hergestellt aus 2 %iger wässriger Lösung).

Das von einem Hund frisch entnommene Blut wird auf drei Teströhrchen verteilt, in welche ca. 0,5 g der Testsubstanz eingewogen wurden. Die Röhrchen werden bei 37 °C gelagert. 3 Minuten nach Entnahme des Blutes wird das erste Röhrchen aus dem Heizblock entnommen, um 90° gedreht und in den Heizblock zurückgestellt. Im Abstand von 30 Sekunden wird der Vorgang wiederholt und die Zeit bis zur Blutgerinnung gemessen. Nachdem das Blut im ersten Röhrchen geronnen ist, wird das nächste Röhrchen überprüft. Als Lee White Clotting Time wird die Zeit definiert, bei welcher das Blut in allen drei Röhrchen geronnen ist. Als Kontrolle wird parallel die Lee White Clotting Time des Blutes ohne Testsubstanz bestimmt. Das Ergebnis ist in Figur 6 graphisch dargestellt und zeigt neben der Lee White Clotting Time für die jeweilige Testsubstanz die Lee White Clotting Time der Kontrolle.

Die Dextranaldehydlösung sowie das Dextranaldehydvlies verkürzen eindeutig die Lee White Gerinnungszeit und zeigen damit die erwartete hämostyptische Wirkung, wobei die reine Dextranaldehydlösung durch die höhere Konzentration und schnellere Verteilung besonders stark die Blutgerinnung beeinflusst. Mit dem Dextranaldehydvlies aus der 2 %igen wässrigen Dextranaldehydlösung konnte die Zeit bis zur Gerinnung des Blutes um ca. 33 % im Vergleich zur Kontrollreaktion ohne Testsubstanz verringert werden.

3. Tierversuch am Schwein (Leber) zur Wirksamkeit des hämostyptischen Vlieses:

Die Wirksamkeit des hämostyptischen Vlies zur Stillung von starken Parenchymblutungen wurde anhand eines Versuchs am Schwein ermittelt. Das hämostyptische Vlies wurde zur Stillung und Versorgung von Leberresektionen und Kreuzschnitten an der Leber eingesetzt.

a) Leberresektionen

Die Blutversorgung der Leber wurde vor der Resektion temporär durch das Pringle-Manöver unterbrochen, bei welchem die Gefäße im Ligamentum hepatoduodenale abgeklemmt werden. Die Gefäße im zu resezierenden Leberlappen wurden mit einer Parenchymklemme abgeklemmt und anschließend die Resektion mittels monopolarer Kauterisation (HF-Chirurgie) durchgeführt (Figur 7).
Die voluminösen Blutgefäße wurden mittels Nahtmaterial fixiert und die Wundfläche mit einem feuchten Vlies abgedeckt. Nach Freigabe des Blutflusses war die Wunde ausreichend versorgt und es traten keine Nachblutungen auf (Figur 8).

b) Kreuzschnitt

Die Leberoberfläche wurde mittels Kreuzschnitte (3 cm lang, 0,5 cm tief, Figur 9) traumatisiert. Im Gegensatz zu den Resektionen wurde die Blutversorgung der Leber während der Traumatisierung nicht unterbrochen.
Das Vlies (aus 2 %iger Dextranaldehydlösung lyophilisiert) wurde in trockenem Zustand auf die Schnittwunde gelegt und mit leichtem Druck mittels einer feuchten Kompresse auf die Wunde moduliert. Die Blutung kam zum Stillstand und die Wunde war ausreichend versorgt (Figur 10).

4. Synthese des Spacers zwischen Polymer und Aldehydfunktion (Figur 18)

Der Spacer wird ausgehend von einem n-Carboxyalkylaldehyd 17 synthetisiert. Zum Schutz der Aldehydgruppe wird 17 in einem Überschuss an Ethylenglykol 18 gelöst und in das entsprechende Acetal 19 unter Rückfluss überführt. Die Acetalisierung erfolgt in saurem Milieu, als Katalysator können p-Toluolsulfonsäure oder 85 %ige Phosphorsäure eingesetzt werden. Auf Grund der Gleichgewichtsreaktion kann das Acetal nach Anbindung des Spacers an das Polymer wieder in das Aldehyd übergeführt werden. Im Neutralen und Alkalischen sind die Acetale stabil. Die Überführung der Carboxygruppe in einen aktiven Ester 21 erfolgt in wasserfreiem DMSO mittels Dicyclohexylcarbodiimid (DCC) und N-Hydroxysuccinimid 20 bei Raumtemperatur und pH7. Alternativ ist die Umwandlung der Carboxygruppe mit Thionylhalogeniden ($SOCl_2$ oder $SOBr_2$) in ein Säurechlorid möglich.

5. Anbindung des Spacers an das Polymer und Darstellung der Aldehydfunktion (Figur 19)

Die Anbindung des Spacers 21a in Form eines aktivierten Esters oder eines Säurechlorids an das Polymer 22 wird wiederum in trockenem DMSO unter Abspaltung von N-Hydroxysuccinimid oder des Halogenids durchgeführt. Die Ausbeute kann durch Zugabe einer Base (z.B. Pyridin) erhöht werden. Im letzten Schritt wird Ethylenglykol 18 im Sauren abgespalten, die Aldehydgruppe regeneriert und das Polymer mit Spacer und Aldehydfunktion 24 dargestellt.

6. Zusammensetzung eines Hämostyptikums mit Vernetzer und Weichmacher

Zusammensetzung 1: C 0/70

| | |
|---|---|
| 70 ml Lösung: Dextranaldehyd (DA), Chitosan und Glycerin | |
| molares Verhältnis: Aldehydgruppe (im DA) zu Aminogruppe (Chitosan) | 28 : 1 |
| molares Verhältnis: Aldehydgruppe (im DA) zu Glycerin | 10 : 1 |

In 35 ml bidestilliertem Wasser werden 1,17 g DA (3,34% w/v) und 0,17 g Glycerin (0,49% w/v) gelöst. Parallel hierzu werden 0,15 g (0,432% w/v) Protasan UP 213 CI® (FMC-BioPolymer AS Oslo; Norwegen, Chitosansalz mit Chlorid als Gegenion) in 35 ml bidestilliertem Wasser gelöst. Die beiden Lösungen werden vereinigt (70 ml), geschüttelt, auf Lyophilisationsschalen (150 x 110 mm) gegossen und abschließend lyophilisiert. Die Dicke der Vliese nach der Lyophilisation beträgt 3,5 mm. Zur Verbesserung der Elastizität werden die Vliese mittels einer Pressvorrichtung auf eine Dicke von 0,3-0,4 mm gepresst.

7. Zusammensetzung eines Hämostyptikums mit Vernetzer, Weichmacher und Zusatz zur Verbesserung der Saugkraft

Zusammensetzung 2: C 5/70

| | |
|---|---|
| 70 ml Lösung: Dextranaldehyd (DA), Chitosan, Glycerin und Carboymethycellulose (CMC) | |
| molares Verhältnis: Aldehydgruppe (im DA) zu Aminogruppe (Chitosan) | 28 : 1 |
| molares Verhältnis: Aldehydgruppe (im DA) zu Glycerin | 10 : 1 |
| molares Verhältnis Aldehydgruppe (im DA) zu Monomer-CMC-Einheit | 1000 : 5 |

In 32,8 ml bidestilliertem Wasser werden 1,17 g DA (3,56% w/v) und 0,17 g Glycerin (0,52% w/v) gelöst. Parallel hierzu werden 0,15 g (0,432% w/v) Protasan UP 213 CI® in 35 ml und 22 mg CMC (1 % w/v) in 2,2 ml bidestilliertem Wasser gelöst. Die Lösungen werden vereinigt (70 ml), geschüttelt, auf Lyophilisationsschalen (150 x 110 mm) gegossen und abschließend lyophilisiert. Die Dicke der Vliese nach der Lyophilisation beträgt 3,5 mm. Zur Verbesserung der Elastizität werden die Vliese mittels einer Pressvorrichtung auf eine Dicke von 0,3-0,4 mm gepresst.

8. Eigenschaften und Anwendung im tierversuch der Vliese C 0/70 und C 5/70

Der Quellungsgrad der Vliese wird wie folgt bestimmt:

Die Vliese werden im gepressten und ungepressten Zustand auf eine Größe von 20 x 20 mm zugeschnitten, ihr Trockengewicht ($W_{tr}$) bestimmt und in 100 ml Sörensen Pufferlösung (pH 7,4) für 5 s getaucht. Nach einer Abtropfzeit von 10 Sekunden wird das Nassgewicht ($W_{aq}$) bestimmt. Der Quellungsgrad lässt sich nach folgender Formel berechnen.

$$Q[\%] = \frac{W_{aq} - W_{tr}}{W_{tr}} \times 100$$

Tabelle 2: Quellungsgrade der Vliese C 0/70 und C 5/70 in gepresstem und ungepressten Zustand

| Produkt | Quellungsgrad |
|---|---|
| C 0/70 ungepresst | 2901 % |
| C 0/70 gepresst | 1688 % |
| C 5/70 ungepresst | 4124 % |
| C 5/70 gepresst | 1946 % |

CMC verbessert die Saugkraft der Vliese und erleichtert somit das Anmodellieren während der OP. Durch das Pressen nimmt der Quellungsgrad in Wasser etwas ab, jedoch ist er mit 1700-2000 % immer noch sehr hoch und zur Blutstillung stark durchbluteter Gewebe ausreichend.

Die Wirksamkeit der gepressten Vliese C 0/70 und C 5/70 wurde anhand der Stillung von diffusen Parenchymblutungen an Ratten überprüft und mit der Wirksamkeit von Lyostypt® (Kollagenvliese B/Braun Aesculap Tuttlingen) verglichen.

Die Traumatisierung der Leber erfolgte mittels einer 1,5 cm langen und 0,3 cm tiefen Kreuzinzision (Abb. 21). Die Polysaccharidvliese wurden in trockenem Zustand auf die Wunde aufgelegt und mit Hilfe einer in physiologischer Kochsalzlösung getränkten Kompresse auf das Gewebe modelliert (Abb. 23). Lyostypt wurde (gemäß Gebrauchanweisung) trocken mit manuellem Druck auf die Wunde aufgelegt. Alle drei Prüfgegenstände hafteten sehr gut auf dem Gewebe und ließen sich nicht mehr durch den Operateur verschieben (Abb. 23). Ein Vergleich der Zeit vom Auflegen des Prüfgegenstandes bis zur Stillung der Blutung bestätigt (vgl. Tabelle 3) die hohe Effektivität der Polysaccharidvliese. Alle insgesamt 27 Tiere (9 Tiere je Produkt) überlebten die OP. Spätestens 4 Tage nach der OP zeigten alle Tiere normales Verhalten.

Die Sektionsbefunde nach 7, 14 bzw. 21 Tagen weisen einen guten Heilungsverlauf der Wunde bei allen Tieren auf. Die Prüfgegenstände deckten das Trauma ab. Bei den Polysaccharidvliesen traten im Gegensatz zu Lyostypt keine Verklebungen der Leberlappen auf, so dass diese auch unerwünschten Adhäsionen vorbeugen. Nach 14 Tagen waren Gefäßeinsprossungen in den Polysaccharidvliesen zu erkennen.

Die makroskopischen Sektionsbefunde wurden durch histopathologische Untersuchungen bestätigt (Abb. 24). Bereits 7 Tagen p. o. wanderten bindegewebige Zellschichten in die Polysaccharidvliese ein. Der Wundspalt war sehr gut verheilt, neues Leberregenerationsgewebe bildete sich bereits innerhalb der ersten 7 Tage.

Tabelle 3: Vergleich der Zeiten vom Auflegen der Hämostyptika bis zur Stillung der Blutung (jeweils 9 Versuchstiere pro Produkt).

| Produkt | Zeit bis zur Blutstillung |
|---|---|
| C 5/70 | 32 s |
| C 0/70 | 38 s |
| Lyostypt® | 62 s |

**Patentansprüche**

1. An menschlichem oder tierischem Gewebe selbsthaftendes resorbierbares Hämostyptikum, im wesentlichen aus mindestens einem, freie Aldehydgruppen tragenden Polymer, dessen Aldehydgruppen mit nukleophilen Gruppen des Gewebes zu reagieren vermögen, wobei das Hämostyptikum in fester poröser und saugfähiger Form vorliegt und es sich bei dem Aldehydgruppen tragenden Polymer um ein oxidiertes Polysaccharid handelt, wobei es sich bei dem oxidierten Polysaccharid um Dextranpolyaldehyd handelt, **dadurch gekennzeichnet, dass** der Anteil an zum Aldehyd oxidierten Glucoseeinheiten im Dextranpolyaldehyd mindestens 20 % beträgt und dass es teilweise mit einem Vernetzungsmittel vernetzt ist.

2. Hämostyptikum nach Anspruch 1, **dadurch gekennzeichnet, daß** es in Form eines dreidimensionalen Körpers, insbesondere einer Platte, vorliegt.

3. Hämostyptikum nach Anspruch 1 oder 2, **dadurch gekennzeichnet, daß** es in Form eines, insbesondere dreidimensionalen, Vlieses vorliegt.

4. Hämostyptikum nach Anspruch 1 oder 2, **dadurch gekennzeichnet, daß** es in Form eines offenporigen Schaumes vorliegt.

5. Hämostyptikums nach Anspruch 1, **dadurch gekennzeichnet, daß** es in Form eines Granulats oder Pulvers aus saugfähigen Partikeln vorliegt.

6. Hämostyptikum nach einem der vorhergehenden Ansprüche, **dadurch gekennzeichnet, daß** der Dextranpolyaldehyd, vorzugsweise das ganze Hämostyptikum, wasserlöslich ist.

7. Hämostyptikum nach einem der vorhergehenden Ansprüche, **dadurch gekennzeichnet, daß** der Anteil an zum Aldehyd oxidierten Glucoseeinheiten im Dextranpolyaldehyd mindestens 35 % bis 100 %, insbesondere 60 % bis 80 %, beträgt.

8. Hämostyptikum nach einem der vorhergehenden Ansprüche, **dadurch gekennzeichnet, dass** es durch Lyophilisierung einer Lösung des Dextranpolyaldehyds erhältlich ist.

9. Hämostyptikum nach einem der vorhergehenden Ansprüche, **dadurch gekennzeichnet, dass** es aus einer 0,5 - 20 %igen, vorzugsweise 1 - 15 %igen, insbesondere 1-10 %igen, bevorzugt 2 %igen, Lösung des Dextranpolyaldehyds erhältlich ist.

10. Hämostyptikum nach einem der vorhergehenden Ansprüche, **dadurch gekennzeichnet, dass** es aufgrund seines hydrophilen Charakters und seiner Porosität mindestens das 30fache seines Gewichtes an Flüssigkeit aufzunehmen vermag.

11. Hämostyptikum nach einem der vorhergehenden Ansprüche, **dadurch gekennzeichnet, dass** es sich bei dem Vernetzungsmittel um mindestens eines aus der Gruppe bestehend aus Chitosan, bi- oder multifunktionellen Aminen, bi- oder multifunktionellen Molekülen mit -SH und -NH$_2$ Gruppen und bi- oder multifunktionellen Thiolen, vorzugsweise um Chitosan, handelt.

12. Hämostyptikum nach einem der vorhergehenden Ansprüche, **dadurch gekennzeichnet, dass** es mindestens einen Zusatz zur Erhöhung der Saugkraft enthält.

13. Hämostyptikum nach Anspruch 12, **dadurch gekennzeichnet, dass** es sich bei dem Mittel zur Erhöhung der Saugkraft um Carboxymethylcellulose (CMC) handelt.

14. Hämostyptikum nach einem der vorhergehenden Ansprüche, **dadurch gekennzeichnet, dass** es eine mindestens einseitig strukturierte Oberfläche ausweist.

15. Verfahren zur Herstellung eines Hämostyptikums gemäss den Ansprüchen 1 bis 14, **dadurch gekennzeichnet, dass** mindestens ein sich in Lösung befindliches und/oder sich im Gelzustand befindliches Dextranpolyaldehyd, das teilweise mit einem Vernetzungsmittel vernetzt ist, mittels Lyophilisation in eine feste trockene Form überführt wird.

16. Hämostyptikum nach einem der Ansprüche 1 bis 14 für eine vorzugsweise innere Anwendung in einem Organismus, insbesondere in Wunden.

17. Hämostyptikum nach einem der Ansprüche 1 bis 14 für den Wundverschluss vorzugsweise innerer Wunden.

**18.** Hämostyptikum nach einem der Ansprüche 1 bis 14 zur Blutstillung bei einer Organresektion oder -ruptur.

**19.** Hämostyptikum nach einem der Ansprüche 1 bis 14 in Form eines Ringes für Anastomosen.

**Claims**

**1.** Reabsorbable hemostyptic self-adhering to human or animal tissue and essentially consisting of at least one polymer which carries free aldehyde groups and whose aldehyde groups are able to react with nucleophilic groups of the tissue, the hemostyptic being present in solid, porous and absorbent form and the polymer carrying aldehyde groups being an oxidized polysaccharide, the oxidized polysaccharide being dextran polyaldehyde, **characterized in that** the proportion of glucose units oxidized to the aldehyde in the dextran polyaldehyde is at least 20% and **in that** it is partially cross-linked with a cross-linking agent.

**2.** Hemostyptic according to Claim 1, **characterized in that** it is present in the form of a three-dimensional body, in particular a sheet.

**3.** Hemostyptic according to Claim 1 or 2, **characterized in that** it is present in the form of a nonwoven, in particular a three-dimensional nonwoven.

**4.** Hemostyptic according to Claim 1 or 2, **characterized in that** it is present in the form of an open-cell foam.

**5.** Hemostyptic according to Claim 1, **characterized in that** it is present in the form of a granulate or powder of absorbent particles.

**6.** Hemostyptic according to one of the preceding claims, **characterized in that** the dextran polyaldehyde, preferably the entire hemostyptic, is water-soluble.

**7.** Hemostyptic according to one of the preceding claims, **characterized in that** the proportion of glucose units oxidized to the aldehyde in the dextran polyaldehyde is at least 35% to 100%, in particular 60% to 80%.

**8.** Hemostyptic according to one of the preceding claims, **characterized in that** it can be obtained by lyophilization of a solution of the dextran polyaldehyde.

**9.** Hemostyptic according to one of the preceding claims, **characterized in that** it can be obtained from a 0.5 - 20% strength, preferably 1 - 15% strength, in particular 1 - 10% strength, especially 2% strength solution of the dextran polyaldehyde.

**10.** Hemostyptic according to one of the preceding claims, **characterized in that**, because of its hydrophilic character and its porosity, it is able to take up at least 30 times its weight of fluid.

**11.** Hemostyptic according to one of the preceding claims, **characterized in that** the cross-linking agent is at least one from the group comprising chitosan, bifunctional or multifunctional amines, bifunctional or multifunctional molecules with -SH and -NH$_2$ groups, and bifunctional or multifunctional thiols, preferably chitosan.

**12.** Hemostyptic according to one of the preceding claims, **characterized in that** it contains at least one additive for increasing the absorbency.

**13.** Hemostyptic according to Claim 12, **characterized in that** the agent for increasing the absorbency is carboxymethylcellulose (CMC).

**14.** Hemostyptic according to one of the preceding claims, **characterized in that** it has a surface structured at least on one side.

**15.** Method for producing a hemostyptic according to Claims 1 to 14, **characterized in that** at least one dextran polyaldehyde in solution and/or in the gel state and which is partially cross-linked with a cross-linking agent, is converted by means of lyophilization into a solid dry form.

**16.** Hemostyptic according to one of Claims 1 to 14, for a preferably internal application in an organism, in particular in wounds.

**17.** Hemostyptic according to one of Claims 1 to 14, for wound closure, preferably of internal wounds.

**18.** Hemostyptic according to one of Claims 1 to 14, for hemostasis in cases of organ resection or organ rupture.

**19.** Hemostyptic according to one of Claims 1 to 14, in the form of a ring for anastomoses.

## Revendications

**1.** Hémostatique résorbable autoadhésif sur un tissu humain ou animal, constitué pour l'essentiel d'au moins un polymère portant des groupes aldéhyde libres, dont les groupes aldéhyde sont à même de réagir avec des groupes nucléophiles du tissu, l'hémostatique se présentant sous forme solide poreuse et absorbante, et dans lequel, pour ce qui est du polymère portant des groupes aldéhyde, il s'agit d'un polysaccharide oxydé, où, pour ce qui concerne le polysaccharide oxydé, il s'agit de dextran-polyaldéhyde, **caractérisé en ce que** la proportion des unités de glucose oxydées en aldéhyde dans le dextran-polyaldéhyde est d'au moins 20 %, et qu'il est partiellement réticulé avec un agent de réticulation.

**2.** Hémostatique selon la revendication 1, **caractérisé en ce qu'**il se présente sous forme d'un corps tridimensionnel, en particulier d'une plaque.

**3.** Hémostatique selon la revendication 1 ou 2, **caractérisé en ce qu'**il se présente sous forme d'un non-tissé, en particulier tridimensionnel.

**4.** Hémostatique selon la revendication 1 ou 2, **caractérisé en ce qu'**il se présente sous forme d'une mousse à alvéoles ouverts.

**5.** Hémostatique selon la revendication 1, **caractérisé en ce qu'**il se présente sous forme d'un granulé ou d'une poudre de particules absorbantes.

**6.** Hémostatique selon l'une des revendications précédentes, **caractérisé en ce que** le dextran-polyaldéhyde, de préférence la totalité de l'hémostatique, est soluble dans l'eau.

**7.** Hémostatique selon l'une des revendications précédentes, **caractérisé en ce que** la proportion des unités de glucose oxydées en aldéhyde, dans le dextran-polyaldéhyde, est d'au moins 35 à 100 %, en particulier de 60 à 80 %.

**8.** Hémostatique selon l'une des revendications précédentes, **caractérisé en ce qu'**il peut être obtenu par lyophilisation d'une solution du dextran-polyaldéhyde.

**9.** Hémostatique selon l'une des revendications précédentes, **caractérisé en ce qu'**il peut être obtenu à partir d'une solution du dextran-polyaldéhyde à 0,5-20 %, de préférence à 1-15 %, en particulier à 1-10 %, de préférence à 2 %.

**10.** Hémostatique selon l'une des revendications précédentes, **caractérisé en ce que**, du fait de son caractère hydrophile et de sa porosité, il peut absorber au moins trente fois son poids de liquide.

**11.** Hémostatique selon l'une des revendications précédentes, **caractérisé en ce que**, pour ce qui concerne l'agent de réticulation, il s'agit d'au moins un agent de réticulation du groupe consistant en le chitosan, les amines bi- ou multifonctionnelles, les molécules bi- ou multifonctionnelles ayant des groupes -SH et -NH$_2$, et les thiols bi- ou multifonctionnels, de préférence du chitosan.

**12.** Hémostatique selon l'une des revendications précédentes, **caractérisé en ce qu'**il contient au moins un additif pour augmenter le pouvoir absorbant.

**13.** Hémostatique selon la revendication 12, **caractérisé en ce que**, pour ce qui concerne le produit pour augmenter le pouvoir absorbant, il s'agit de carboxyméthylcellulose (CMC).

**14.** Hémostatique selon l'une des revendications précédentes, **caractérisé en ce qu'**il présente une surface structurée au moins sur une face.

**15.** Procédé de fabrication d'un hémostatique selon les revendications 1 à 14, **caractérisé en ce qu'**un dextran-polyaldéhyde, qui est partiellement réticulé par un agent de réticulation, se trouvant en solution et/ou se trouvant à l'état de gel, est converti par lyophilisation en une forme sèche solide.

**16.** Hémostatique selon l'une des revendications 1 à 14, pour une utilisation de préférence interne dans un organisme, notamment dans des plaies.

**17.** Hémostatique selon l'une des revendications 1 à 14, pour la fermeture des plaies, de préférence des plaies internes.

**18.** Hémostatique selon l'une des revendications 1 à 14, pour l'hémostase en présence d'une résection ou d'une rupture d'un organe.

**19.** Hémostatique selon l'une des revendications 1 à 14, sous forme d'un anneau pour anastomoses.

**Figur 1**

**Figur 2**

HV=25.00 kV
Arbeitsabstand= 22 mm
100µm
Vergrößerung= 100 X
Detektor= RRE
Photo Nr.=5

**Figur 3**

**Figur 4**

**Figur 5**

# Figur 6

**Lee White Clotting Time Studie**

**Figur 7**

12

13

**Figur 8**

14

**Figur 9**

15

**Figur 10**

14

Fig. 14

Fig. 13

Fig. 12

Fig. 11

11

Fig 15

Fig 16a

2

2

Fig 16.b

EP 1 615 674 B1

**Figur 17**

16

**Figur 18**

## Figur 19

mit X= Cl, Br, I und N-Hydroxysuccinimid

n= 0-10 bevorzugt 0-4

**Figur 20**

**Bereitstellungsbeispiele für Organresektionen oder - rupturen**

**Milz**

- ○ Milzrisse
- • Splenektomie
- ◎ Milzzysten

**Leber**

- • Traumatische Rupturen
- • Lebertumor
- • Lebermetastasen
- • Leberresektionen

**Bauchspeicheldrüse**

- • Traumatische Rupturen
- • Nesidioplastome
- • Behnadlung vonPseudo-zysten
- • Ausschabung von Tumo-ren

**Niere**

- • Traumatische Rupturen
- • Ausschabung von Tumo-ren
- • Nephrektomie

**Darm**

- ○ Insuffiziente Anastomosen
- • Darmplikaturen

**Lunge**

- • Traumatische Rupturen
- • Tumor/ Metastasen
- • Lungenresektionen
- • Pleurodese

**Laparoskopie/Minimal invasive Chirurgie**

- • Ovarialzysten

**Prostata**

- • Prostatektomie
- • Prostatatumor

**Figur 21**

**Figur 22**

**Figur 23**

Figur 24

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6156488 A
- DE 10152407
- EP 815879 A
- EP 693291 A
- EP 1424085 A1
- US 3868955 A

**In der Beschreibung aufgeführte Nicht-Patentliteratur**

- **OKUYAMA H. et al.** Laparoscopic rectopexy for rectal prolapse in children. *Pediatric Endosurgery and Innovative Techniques,* 2002, vol. 6 (4), 285-288